# EUROPEAN PATENT APPLICATION

(11) **EP 4 372 752 A1**
(43) Date of publication of application: **22.05.2024**
(21) Application number: 22208631.6
(22) Date of filing: 21.11.2022
(51) Int. Cl.: G16H 15/00, G16H 80/00, G16H 10/65, G16H 40/63, G16H 40/67, G16H 50/20

(54) **CARDIAC TELEMETRY SYSTEM WITH SYMPTOM DESCRIPTION AND METHOD OF OPERATION THEREOF**

(71) Applicant: Koninklijke Philips N.V., 5656 AG Eindhoven (NL)
(72) Inventor: STUT, Wilhelmus Johannes Joseph, 5656AG Eindhoven (NL); TEN KATE, Warner Rudolph Theophile, 5959AG Eindhoven (NL); DE JAGER, Marinus Karel Johannes, 5656AG Eindhoven (NL); GELISSEN, Jozef Hubertus, 5656AG Eindhoven (NL)
(74) Representative: Philips Intellectual Property & Standards

(57) **Abstract**

A mobile telemetry system (100 including a mobile telemetry unit (MTU) (102) including: a sensor (114) for detecting a physiological signal; and a controller (120) configured to: communicate with a mobile station (MS) (104) to perform a clock synchronization with a clock of the MS (104); obtain the physiological signal from the sensor and form physiological information; determine whether a time stamped transmit information request (TS-XReq) including time stamp information (TS) was received from the MS (104); and generate and transmit physiological information which corresponds in time with the received TS when it is determined that the TS-XReq was received from the MS (104).

## Description

### FIELD OF THE INVENTION

The present system relates to a portable cardiac telemetry system to record electrocardiogram (ECG) signals of patients and, more particularly, to a portable cardiac telemetry system to record ECG signals and a symptom description, and a method of operation thereof.

### BACKGROUND OF THE INVENTION

Holter monitors and mobile cardiac telemetry systems typically gather ECG signals in ambulatory settings (e.g., in the home environment or on-the-go) from a patient. In operation, the patient typically places a sensor, connected to a patch, on their chest. The sensor communicates wirelessly with a dedicated smartphone which is connected (via a mobile phone network) to a clinical service center.

The sensor may measure electrocardiogram (ECG) signals continuously (e.g., 24 hours a day, seven days a week) with only a subset of the ECG signals being sent to the clinical service center via the mobile phone network. In other systems, the sensor measures the ECG signals periodically and/or when the patient has informed the system that that the patient feels a symptom. In any event, the sensor creates and transmits an ECG strip (e.g., the ECG signals captured over a period of time, such as 90 seconds) to the dedicated smartphone which subsequently sends the ECG strip(s) to the clinical service center. In a case when the patient has informed the system that the patient feels a symptom, the symptom description, as selected from a menu by the patient on the smartphone, is also sent to the clinical service center.

In some systems, when a symptom is felt, the patient may inform the sensor about the occurrence of the symptom by touching the sensor in a specific way. For example, the patient may double tap on some Philips^{™} BioTel^{™} sensors, whereas Medicomp^{™} offers a dedicated symptom button on the sensor. Touching the sensor in the specific way adds a mark to the ECG data stream and instructs the sensor to create a 90 second ECG strip. Note that in these prior systems, by touching the sensor, the sensor only registers that a symptom has occurred, yet the patient has not described the symptom.

The description of symptoms is typically done via an application (app) on the smartphone. When a symptom is felt, the patient may select the symptom recording function of the app and provide details, like the nature of the symptom (e.g., feeling faint, dizzy, chest pain, etc.), the activity and/or intensity of the activity during which the symptom occurred and the location of the patient at the time that the symptom occurred. In addition, the current date and time are recorded together with the ECG strip.

Some systems also offer voice recording. For example, in the Medicomp^{™} TelePatch^{™} system, the patient may choose whether the symptom details are to be entered manually or via audio by manually pressing a corresponding button in the smartphone app. Once the data of the symptom event (i.e., the ECG strip and description) have been received by the clinical service center, an ECG technician may interpret the received ECG signal and symptom description, and act accordingly (e.g., call the patient or a physician).

One of the drawbacks of the prior solutions is that in order to describe their symptoms in the app, the patients must look at and physically interact with (e.g., press) the smartphone screen. In some cases, this may not be desired, possible, or allowed (e.g., when driving a car, operating machinery, etc.). The inability to interact with the smartphone at the time of a symptom may result in the loss of valuable data which may have been used by a clinician or doctor for diagnostic purposes and to provide proper medical treatment. Another drawback of the prior solutions is that the patient may only describe the symptom when the phone is in reach and working properly. In a case wherein the patient travels without the phone, or the phone battery is depleted, the symptom details may not be entered. This again may result in the loss of information reducing the amount of information available for proper diagnosis and treatment of the patient. This drawback also has the added negative effect in that ECG strips associated with the patient feeling and describing a symptom are particularly useful in evaluating the patient's condition and yet, it is just this type of valuable information that may be lost by prior systems or otherwise absent from the information that is evaluated.

To overcome the aforementioned barriers and detriments as well as others, there is a need for a mobile telemetry system (e.g., mobile cardiac telemetry system) and method of operation thereof that may effectively provide a patient's physiological signals (e.g., ECG signals) and symptom descriptions for events that occur even when manipulation of the mobile unit (e.g., smartphone) is not convenient or possible. Accordingly, embodiments of the present system may overcome these and other disadvantages of conventional mobile telemetry systems.

### SUMMARY OF THE INVENTION

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a mobile telemetry system including a mobile telemetry unit (MTU). The MTU may include: a sensor for detecting a physiological signal; and a controller configured to: communicate with a mobile station (MS) to perform a clock synchronization with a clock of the MS; obtain the physiological signal from the sensor and form physiological information; determine whether a time stamped transmit information request (TS-XReq) including time stamp information (TS) was received from the MS; and generate and transmit physiological information which corresponds in time with the received TS when it is determined that the TS-XReq was received from the MS. The controller of the MTU may be a first controller with the system further including the MS. The MS may have a second controller configured to transmit the TS-XReq to the MTU and to control a voice recording mode (VRM) to obtain symptom information corresponding to the TS of the TS-XReq and form corresponding user response information (URI). The second controller may be configured to associate the TS with the URI to form time stamped user response information (TS-URI).

In accordance with embodiments, it is disclosed that the second controller may be configured to generate the time stamp information (TS) and the TS-XReq in response to a user interaction at the MS (104) and to transmit the TS-XReq to the MTU. The second controller may be configured to control a voice recording mode (VRM) to obtain symptom information corresponding to the TS of the TS-XReq and form corresponding user response information (URI). The second controller may be configured to associate the TS with the URI to form time stamped user response information (TS-URI). The second controller may be further configured to associate corresponding patient identification information (ID) with the generated TS-URI to form ID-TS-URI. The first controller (120) may be configured to transmit the physiological information to the MS. The second controller may be further configured to transmit the ID-TS-URI and the physiological information to a clinical service center (CSC).

In accordance with embodiments, it is disclosed that the second controller (190) may be configured to control a voice recording mode (VRM) to obtain symptom information corresponding to the TS of the TS-XReq and form corresponding user response information (URI) associated with the TS as time stamped user response information (TS-URI). The MS may be a first MS, the sensor of the MTU may be a first sensor, and the system may include a second MS having the second controller. The second controller may be further configured to receive the TS-XREQ, the first MS having a third controller and a second sensor for detecting a user interaction, wherein the third controller may be configured to determine whether a user interaction is detected by the second sensor and transmit the TS-XREQ to the second MS and to the MTU when the user interaction is detected. The second controller may be further configured to associate corresponding patient identification information (ID) with the generated TS-URI to form ID-TS-URI.

The first controller may be configured to transmit the physiological information to the MS, and the second controller may be further configured to transmit the ID-TS-URI and the physiological information to a clinical service center (CSC). In another embodiment wherein the sensor of the MTU is a first sensor, the MTU may further include a second sensor for detecting a user interaction, wherein the controller may be configured to generate and transmit, when it is determined that the second sensor has detected the user interaction: physiological information which corresponds in time with when the user interaction was detected by the second sensor; a TS which corresponds in time with when the user interaction was detected by the second sensor; and a TS activate voice recording mode (TS-VRM) command configured to activate a voice recording mode (VRM) of a mobile station to capture user response information (URI).

In accordance with embodiments of the present system, it is disclosed that a mobile telemetry system may include a mobile telemetry unit (MTU) including: a first sensor for detecting a physiological signal; a second sensor for detecting a user interaction; and a controller configured to: communicate with a mobile station (MS) to perform a clock synchronization with a clock of the MS; determine whether the user interaction is detected by the second sensor; and generate and transmit, when it is determined that the second sensor has detected the user interaction: physiological information from the physiological signal which corresponds in time with when the user interaction was detected by the second sensor; a TS which corresponds in time with when the user interaction was detected by the second sensor; and a TS activate voice recording mode (TS-VRM) command configured to activate a voice recording mode (VRM) of the MS to capture user response information (URI).

The controller of the MTU may be a first controller (120), with the system further including the mobile station having a second controller configured to receive the TS-VRM command and in response, control the VRM to obtain symptom information corresponding to the TS-VRM received from the MTU and form corresponding user response information (URI). The second controller may be configured to associate the received TS from the TS-VRM with the URI to form time stamped user response information (TS-URI). The second controller may be further configured to associate corresponding patient identification information (ID) with the generated TS-URI to form ID-TS-URI. The first controller may be configured to transmit the physiological information to the MS, and the second controller may be further configured to transmit the ID-TS-URI and the physiological information to a clinical service center (CSC).

Further, in accordance with embodiments of the present system, it is disclosed that a mobile cardiac telemetry unit (MCTU) may include a cardiac sensor for detecting cardiac signals and a controller configured to: obtain cardiac information from the plurality of cardiac sensors and form electrocardiograph information (ECGI); determine whether a user request has been generated and form time stamp information (TS) when it is determined that the user request has been generated, the TS substantially corresponding with a time that the user request was determined to be generated; form an activate voice recording mode command (AVRM) configured to activate a voice recording mode (VRM) of a mobile station (MS) to capture user response information (URI); generate electrocardiograph information (ECGI), which corresponds in time with the TS; and transmit the TS, the AVRM and the ECGI. The cardiac sensor (114) may be a first sensor with the system further including a second sensor for detecting a user interaction. The controller may be configured to: determine whether the user interaction is detected by the second sensor, the user interaction indicating the user request; and transmit the TS, the AVRM and the ECGI when it is determined that the second sensor has detected the user interaction.

### BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:
FIG. 1 shows an illustrative schematic block diagram of a portion of a system operating in accordance with embodiments of the present system;
FIG. 2 shows an illustrative functional flow diagram performed by a process in accordance with embodiments of the present system; and
FIG. 3 shows an illustrative functional flow diagram performed by a process in accordance with embodiments of the present system.

### DETAILED DESCRIPTION OF EMBODIMENTS

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or" and formatives thereof, should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", "substantially" and "approximately" denote an interval of accuracy that a person skilled in the art will understand to still ensure the technical effect of the feature in question which in some cases may also denote "within engineering tolerances." For example, the terms may indicate a deviation from the indicated numerical value(s) of ±20 %, ±15 %, ±10 %, ±5 %, ±1 % ±0.5 % or ±0.1 %.

The terms clinician, clinicians, or formatives thereof, may include a professional such as doctor, a clinician, an operator, an expert, a technician, and/or the like, who may review information related to the patient and/or other information transmitted by embodiments of the present system. The terms patient, patients, or formatives thereof, may include patients or other individuals (e.g., subjects, etc.) or other users who may be receiving/using any type of cardiac monitoring systems, machines, processes, and/or methods operating in accordance with embodiments of the present system. In addition, though electrocardiograms, electrocardiogram information (ECGI), etc., is illustratively discussed herein to simplify the following description, it should be understood that other physiological information may be sensed (e.g., captured, measured, determined, etc.), processed, transmitted, annotated, etc., in accordance with embodiments of the present system. For example, other vital signs may be detected by a sensor of the system, such as arrythmias, oxygen saturation (SpO2) levels, etc. In fact, any detectable symptom(s) relevant for the purpose of a wearable or other device being used for observation may be sensed, etc., in accordance with the present system. Accordingly, the discussion herein regarding the present system and electrocardiograms (ECG), ECGI, etc., should be understood to similarly apply to one or more (e.g., two or more may be sensed) of this other physiological information without limitation.

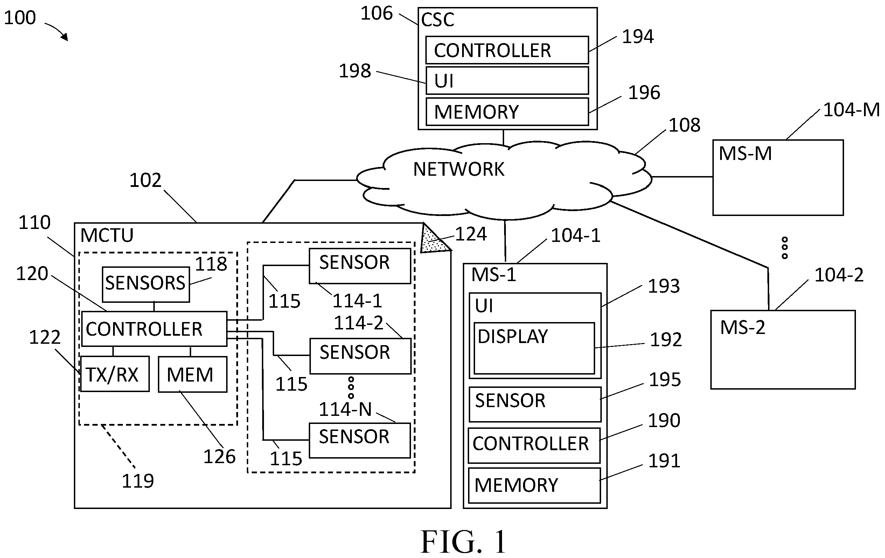

FIG. 1 illustratively shows a schematic block diagram which shows a portion of a system 100 (hereinafter the system 100) operating in accordance with embodiments of the present system. The system 100 may include one or more of a mobile telemetry unit (e.g., a mobile cardiac telemetry unit (MCTU)) 102, such as body-worn device, one or more mobile stations (MS) 104-1 through 104-M (where M is an integer), such as an off-body device, and a clinical service center (CSC) 106, one or more of which may communicate with the other via any suitable communication method or methods such as wired and/or wireless (e.g., RF-based, optical, etc.) communication methods over a network 108. While a mobile cardiac telemetry system (MCTU) is illustratively referenced to simplify the following discussion, it is understood that the MCTU 102 may in fact be any mobile telemetry unit in accordance with embodiments of the present system. Accordingly, the following discussion and claims herein should be understood to encompass a cardiac monitoring system and/or other physiological monitoring systems (e.g., mobile telemetry unit), without limitation unless one or more given systems are explicitly excluded. The network 108 may include any suitable network such as an ad-hoc network, a body area network (BAN), a personal area network (e.g., e.g., Bluetooth^{™}, etc.), a Wi-Fi network, a local area network (LAN), a wide area network (WAN), a telephony network, a cellular network, a satellite connection, etc.

While more than one MS 104-x may be present in accordance with embodiments of the present system, for the sake of clarity, only a single MS 104-x will be discussed, further referred to generally as MS 104, unless the context indicates otherwise. The discussions herein related to the MS 104 should be understood to at least optionally apply to any one or more of the MS 104-x.

The MCTU 102, the MS 104, the CSC 106 and the network 108 may be controlled by at least one controller (e.g., one or more controllers) which may control the overall operation of the system and may include one or more logic devices such as a microprocessor (µP) and/or the like, typically coupled to a memory. The controller may control the overall operation of the system 100. It should be appreciated that in some embodiments the controller may include digital and/or analog control circuitry. The memory may be any type of device for storing application data as well as other data related to the described operation such as application data, operating parameters, user supplied information, sensor measurements, etc., and/or combinations thereof. The application data, operating parameters, user supplied information, sensor measurements, etc., and/or combinations thereof, may be received by the controller for configuring (e.g., programming) the controller to perform operation acts in accordance with the present system. The controller so configured becomes a special purpose machine particularly suited for performing in accordance with embodiments of the present system.

The at least one controller may access the memory and may obtain system setting information (SSI) which may include one or more of operating parameters (e.g., a time duration of ECG signals, etc.), threshold values, warnings, display settings, user information, patient information such as patient identification (ID), etc. The SSI may be set by the system and/or a user and may be updated during use. Further, the system under control of the controller may generate and render an interface with which the patient and/or clinician may, for example, interact to set, reset, select, and/or adjust one or more settings of the SSI. The controller may control the rendering of content, such as still or video information, as well as audio information on a rendering device of the system such as on a display (e.g., display 192) and/or speaker of a user interface (e.g., UI 193). This information may include information related to application data, operating parameters, user supplied information, sensor measurements, instructions, feedback, and/or other information related to an operation of the system or portions thereof such as SSI or portions thereof, clinical decision support, application data, time stamp information, ECG information, etc., and/or combinations thereof.

The MS 104 may include any suitable device, such as a mobile station, smartphone (e.g., an I-Phone, a Galaxy phone, etc.), personal digital assistant (PDA), a smart watch (e.g., an Apple

Watch^{™}, a Galaxy^{™} watch, etc.), smart ring, smart headband, etc. (generally token) and/or the like. The MS 104 may include one or more of a memory 191, at least one controller 190, a user interface (UI) 193, a sensor 195 and a transceiver (e.g., a transmission/reception device) (Tx/Rx) configured to communicate with the network 108 to, for example, be coupled to one or more of the MCTU 102, the CSC 106 and one or more other MSs. As such, more complicated and simpler configurations of the MS are contemplated herein. For example, an MS such as a smart ring and/or other token, may lack a display, yet be able to operate in accordance with other operations described herein.

The UI 193, when present, may include an interface with which a user may interact, such as a touch-screen display 192, an optical sensor such as a camera, a proximity sensor, a speaker, and/or a microphone. The UI 193 may include a user input device such as a keyboard, a mouse, a trackball, touch sensitive sensor, or other device, such as a touch-sensitive display, gyroscope, accelerometer, etc., which may be stand alone or part of a system, such as part of a laptop, a personal digital assistant (PDA), a mobile phone (e.g., a smart phone), a smart watch, token or other device for communicating with the controller 120/190 via any operable link such as a wired and/or wireless communication link. The user input device may be operable for interacting with the controller 120 including enabling interaction within the UI 193 as described herein. The UI 193 may be operative to provide/receive audio/visual information, data, user input such as touch and gesture input, etc., to/from the user of the present system and may inform the patient of operating parameters, operating states, etc.

The UI 193 may render information, such as content, etc., on a speaker and/or display for the convenience of the patient. In addition, the UI 193 may receive information entered by the patient such as selections, requests, commands, gestures, patient settings, etc., using any suitable input method such as gesture inputs, a touch input, voice input, etc. In accordance with embodiments of the present system, the input may be a voice input provided via the microphone such as an audibly recorded user response information (URI) as further described herein. The memory 191 may store information for use by the system such as information received via the UI 193, the SSI, ECG information, time stamp information, the URI, an activate voice recording mode command (AVRM), the patient identification information (ID), system settings and/or system parameters (e.g., SSI), etc., and/or combinations thereof, as described further herein.

The sensor 195 of the MS 104 may be enabled to capture information from the patient, such as one or more of proximity information, touch information, gesture information, physiological information, etc., for operation in accordance with the UI 193 and/or otherwise with the present system. For example, when the MS 104 is a smart watch, the sensor 105 may sense physiological information such as one or more of the patient's cardiac information (ECGI), blood oxygenation, etc., as well as one or more other parameters such as exertion level, altitude, location, etc. Accordingly, in embodiments wherein the MS 104 has one or more of these capabilities, the MS 104 may initiate forming of the URI together with or independent of the MCTU 102 forming the physiological information, such as the ECGI. For example, when the MS 104 has capabilities to capture the ECGI, it may do so in accordance with the present system, without involving or together with the MCTU 102. Further, through the UI 193, the MS 104 may notify the patient to perform an act, such as audibly, visually and/or tactilely informing the patient to place a body part (e.g., fingers) in contact with the sensor 195 to enable capture of the ECGI by the MS 104. In these embodiments, the MS 104 may capture the ECGI and/or other information together with or independent of the MCTU 102 and thereafter form the URI as described herein. In accordance with embodiments of the present system, the MS 104 and/or the MCTU 102 may initiate forming of the URI in response to other information, such as in response to a sensed exertion level of the patient and/or other information that may be sensed by the MCTU 102 (e.g., when the MCTU 102 senses other physiological information together with or in place of the cardiac information). For example, after a strenuous exertion of the patient is sensed by the sensor 195 or a sensor 114, 118, any one of the controllers of the present system may initiate the MS 104 to form the URI by, for example, informing the patient that a strenuous activity or other information of the patient was sensed, and thereafter requesting URI from the patient regarding, for example, how the patient feels currently or felt at that time of the sensed information and thereby, form the URI accordingly.

To simplify the following discussion, physiological information, such as the ECG information, may include physiological information captured over a period of time, such as ECG strips captured over a period of time. To simplify the following discussion, the physiological information captured over a period of time, such as the ECG strips captured over a period of time, will be discussed herein simply as ECGI, though should be understood to apply similarly to other physiological information, activity information, etc., captured over a period of time. Patient symptom and/or context descriptions including audibly recorded patient symptom and/or context descriptions that in accordance with the present system are associated with the physiological information (e.g., ECGI), , activity information, etc., will be referred to herein generally as user response information (URI). The timing information, whether it be an indication of when information is captured and/or a period of time over which the information is captured and that is associated with different information will be referred to herein simply as time stamp (TS).

With regard to the TS, the TS may include time information in any suitable format as may be set forth in system settings as may be stored in the SSI. For example, in some embodiments, the TS may include only time information (e.g., in any suitable format such as in 24-hour format, etc.) while in other embodiments the TS may further include one or more of day and date information. Thus, the TS may include time (e.g., 08:10:25 PM), day (e.g., Wednesday, etc.), and/or date (e.g., 04-20-2020) in any suitable format such as in month, day, year, time, etc. These formats for the TS may be set by the system and/or clinician and may be stored in a memory of the system such as in the SSI for later use. As contemplated herein, these formats for the TS may have a default format as is set without patient or clinician interaction.

In accordance with embodiments of the present system, the ID may be employed to identify a patient using the MS 104 and/or using the MCTU 102. For example, the MS 104 may be associated with an ID or the MS 104 may solicitate the user to identify themselves orally and/or through other interaction with the UI 193. In operation, the MS 104 may transmit information such as operating parameters, instructions, requests, commands (e.g., a synchronization command, etc.), the ECGI, the TS, the URI, etc., to the MCTU 102 and/or the CSC 106 and may receive corresponding information such as time-stamped ECGI (TS-ECGI) from the MCTU 102.

In accordance with embodiments of the present system, terms such as TS-ECGI is intended to convey that time-stamp information (e.g., information indicating a time when information is captured, when the information is started to be captured, and/or the duration of information capture, e.g., starting on May 4, 2022, at 9:10 AM) is associated by the system (e.g., by the MS 104) with a corresponding ECGI. In accordance with embodiments of the present system, the association may be accomplished by embedding one information (e.g., TS) into another type of information (e.g., ECGI); may be accomplished by appending the one information into the other information; may be accomplished by storing the one information and the other information in the same, contiguous or associated data storage locations; and/or may be accomplished by transmitting the one information and the other information in the same, contiguous or associated information streams. Other such terms, such as TS-URI discussed further herein (e.g., user response information (URI) associated with corresponding time stamp information (TS) to form time stamped URI (TS-URI)), should be understood to have a similar connotation. For example, the MS 104 may further interact with the patient to form time-stamped user response information (TS-URI) as described further herein.

The MS 104 may also transmit the TS-URI and/or an associated time stamped ECGI (TS-ECGI) to the CSC 106 for further processing. In addition, the MS 104 may include the (patient) ID with the corresponding transmitted information and thereby form ID-TS-URI and ID-TS-ECGI. In these embodiments, the URI and ECGI are independently associated with the ID and the TS. In accordance with embodiments of the present system, the MS 104 may associate the TS, URI and ECGI together to form TS-URI-ECGI and/or together with the ID to form ID-TS-URI-ECGI. As discussed herein, it is intended that when two or more elements (e.g., URI and ECGI) are said to be each associated with a third element (e.g., TS), each of the two or more elements may be independently associated with the third element and/or the three elements may be simply associated all together (e.g., stored together, transmitted together, referenced by a common reference, stored with a common reference, stored in a given storage location, etc.). Other ways that the elements described herein may be associated are intended to be encompassed by the present system. Accordingly, the discussion herein regarding associated elements is intended to encompass all the suitable ways of associating the elements together.

In accordance with embodiments of the present system, the MCTU 102 may gather information independent of the MS 104, and associate the information together, such as associating the TS with the ECGI to form the TS-ECGI. In these embodiments the MCTU 102 may thereafter forward the TS-ECGI, etc., to the MS 104 for further processing, storage, forwarding, etc. However, for the sake of clarity, many of the actions described herein are described as being performed by one or more of the MS 104, however, unless the context indicates otherwise, these actions (e.g., such as the association of information together) should be understood to also be able to be performed by the MCTU 102 independent of or together with the one or more MS 104.

The MS 104 may further receive information including a request from the MCTU 102 to activate a voice recording mode (VRM) and in response thereto, the MS 104 may activate the VRM. As described herein, the MS 104 may also activate the VRM independent of the MCTU 102. When the MS 104 is in the VRM, the MS 104 may generate and/or render an interface using the UI 193 with which the patient may interact to enter information (e.g., information related to the patient such as symptoms, context, etc.) which may be processed to form corresponding URI and may be further processed to form corresponding TS-URI. For example, when in the VRM, the MS 104 may render a request (e.g., such as an audible request, a displayed request, and/or a tactile request) for the patient to verbally convey symptoms and/or other context (e.g., activity level at a given time) of the patient at a given time and/or time period (e.g., such as corresponding to when an ECGI was captured by the MCTU 102 as indicated by the TS of a received TS-ECGI). In accordance with embodiments of the present system, the symptoms provided by the patient and/or the other context may be recorded by the MS 104 when in the VRM

For the sake of clarity, symptom(s) may be further discussed, however, the related description herein should be understood to also apply to any context supplied by the patient (e.g., symptoms and/or context). Thereafter, the MS 104 may form corresponding URI which may be time-stamped with a corresponding time stamp (e.g., wherein the time stamp may correspond with an event, such as when the ECGI was captured in the past or may correspond with an event at the current time) to form the TS-URI. In accordance with embodiments of the present system, once a symptom is provided by the user and is associated with a corresponding event including TS, ECGI, etc., the MS 104 may store the event information and/or transmit the event information to the CSC 106. Thereafter, the event need not be further processed by the system 100.

In some embodiments, the system 100 may store one or more undescribed events with a corresponding TS-ECGI in a memory of the system 100 (such as at the MCTU 102) for later solicitation of a description of symptoms by the patient (such as by the MS 104). For example, when the MCTU 102 detects an event, such as an irregular heartbeat, and/or the user indicates to the MCTU 102 that an event is occurring, such as one or more symptoms are being felt by the user, but the MCTU 102 is not coupled to the MS 104 or interaction with the MS 104 is not suitable (e.g., when the patient driving), the MCTU 102 may store one or more of the undescribed events with corresponding TS-ECGI(s) in a memory (e.g., store as a list), such as a memory 126. Thereafter, when the MCTU 102 is coupled to (e.g., communication is established or reestablished) the MS 104 subsequently, the MCTU 102 may transmit the one or more undescribed events with the corresponding TS-ECGI to act as a trigger signal, although a separate trigger signal may also be transmitted, to trigger the MS 104 to solicit capture of the URI, such as symptom description(s), for storing, transmitting, etc., together with the event information of the one or more previously undescribed events. Thus, if undefined events occur over a period, such as one or more hours, days, etc. (e.g., such as may occur when the patient may lose or break the MS 104), the MCTU 102 may, upon reestablishing communication with the MS 104, send a signal to the MS 104 to initiate the VRM to obtain the URI for more than one event, day, etc.

When the MS 104 includes a smart watch, token, or the like, the smart watch, etc., may perform the operations of the MS 104 and/or may interact with another MS 104 and/or the MCTU 102 to indicate that a symptom is detected (e.g., when the smart watch has one or more symptom detection sensors 195, such as a pulse detector, ECG sensor, etc.). Further, the smart watch, etc., and/or sensor thereof, may be utilized to receive an input from the user, such as in a form of a chopping gesture, speech, touch, etc., to indicate that a symptom is being felt by the user. In accordance with embodiments of the present system, MS 104, such as the smart watch, etc., may activate the MCTU 102 to capture an ECGI, TS, etc., for subsequent association, processing, storage, transfer, etc., as described herein.

Accordingly, in some embodiments, the smart watch, etc., MS 104 may have sufficient capabilities to perform all actions necessary by the system such as communication with the MCTU 102, communication with the network 108, and communication with the CSC 106, such as via a cellular network (e.g., via a 5G connection), capturing the ECGI, etc. However, in yet other embodiments, the MS 104 may lack some of these capabilities (e.g., it may lack cellular communication abilities). In an embodiment wherein the MS 104 lacks one or more capabilities, the MS 104 may be employed to perform some actions required by embodiments of the present system and may rely upon one or more other MS 104 for other actions, such as cellular communication capabilities such as may be provide by the MS 104-1. For example, a smart watch, etc., MS 104-2 may, for example, be employed to enter a gesture or touch request by the patient to, for example, indicate that a symptom is felt by the patient. Thereafter, the smart watch, etc., MS 104-2 may transmit corresponding information to the MCTU 102 and/or the MS 104-1, which one or more may use this information during one or more operations that may be performed. Further, the smart watch, etc., 104-2 may be considered an extension of the MS 104-1 and may extend the capabilities of the MS 104-1 using remote sensors such as gyroscope, accelerometer, UI, etc., to sense, for example, a gesture input (e.g., chopping input) by the patient and may transmit information of such to the MS 104-1 for further processing.

The system and/or user may determine which information may be employed from which MS 104 and information on such may be stored in the SSI. For example, the smart watch, etc., MS 104-2 may be employed to enter gestures while the MS 104-1 may be employed for cellular communication with the CSC 106 via a cellular network. Settings indicating which MS 104 may perform which operative actions in accordance with embodiments of the present system may be set by the system, clinician, and/or patient and may be stored in the SSI for later use. Thus, for example, a patient may set settings such that the smart watch, etc., MS 104-2 may be employed to enter gestures which may be forwarded to the MS 104-1 for further processing, such as forming the ECGI, forwarding a request to the MCTU 102 to capture the ECGI, forming the URI, etc. While a smartwatch is illustratively described, other MS may be employed such as smart ring (generally token) and may operate similarly. For example, the patient may perform a gesture with the smart ring, such as shaking the smart ring to indicate that a symptom is being experienced. Although, one or more of the MS 104 may be enabled to perform all operations of the MS 104 in accordance with embodiments of the present system.

The CSC 106 may include a controller 194, a memory 196, and a UI 198 with which the user may interact. The controller 194 may control the overall operation of the CSC 106 and, as such may receive information, such as the ID-TS-URI and/or ID-TS-ECGI, from, for example, the MS 104, and may further process this information. The controller 194 may include an engine running an artificial intelligence (AI) program, or other form of data & signal processing, to process the received information to determine whether cardiac conditions, such as arrythmias, irregular rhythm, etc. (e.g., via an abnormality detection engine, etc.), are present in one or more received TS-ECGI and may inform a clinician of such by rendering corresponding information on the UI 198, such as a display and/or other rendering device such as a speaker, etc. The clinician may then contact the patient, for example, via one or more of the MS 104 and/or may contact a doctor or other medical professional for further analysis and possible action. For example, the clinician may observe that a received TS-ECGI indicates an abnormal event is indicated in the ECGI (e.g., atrial fibrillation is indicated in the ECGI) and may interact with a user interface to enter and/or select information (e.g., such as information that identifies the abnormality) and/or may select to contact the patient via the patient's MS 104 via a selected method (e.g., set by the patient, and/or the system) such as via a phone call, a text message, an email, a social media account (e.g., WhatsApp, Facebook, etc.), etc. In some embodiments, when certain conditions are detected (e.g., arrhythmias, etc.), the CSC 106 may contact the patient via the selected contact method to solicit whether the patient felt any symptoms at the time of the TS-ECGI so that one or more of the MS 104 and the CSC 106 may form, associate, etc., the URI and other information as described herein.

In accordance with embodiments of the present system, other forms of processing and communicating are considered as part of the present system. For example, physiological information, such as the ECGI, may be labeled or otherwise identified (e.g., with a pointer to the information location in one or more memories and/or stored at a dedicated location in one or more memories). In this way, for example, the information may be grouped (e.g., similarly labeled, grouped, stored, processed, etc.) so that similar events (e.g., tachycardia events) get grouped together to expedite later/further analysis.

The MCTU 102 may include one or more of a controller 120, a Tx/Rx 122, the memory 126, sensor(s) 114-1 through 114-N (generally sensor 114, where N is an integer) and sensor(s) 118, and at least one substrate 110. The sensor 114 (e.g., one or more of) may be an electrical signal sensor, an optical sensor, a pressure sensor, a motion sensor, a sound sensor (e.g., a microphone), and/or one or more other types of suitable sensors for sensing physiological information (e.g., vitals) of a patient and/or that may be used to detect an irregularity or out-of-range value related to the physiological information. For example, in an embodiment wherein the sensor is a Photoplethysmography (PPG) sensor, the PPG sensor may be utilized to evaluate blood flow near the surface of the skin, for example, to detect arrythmias of the patient. As discussed herein, though an electrocardiogram sensor and/or a mobile cardiac telemetry unit is illustratively described to simplify the discussion herein, the discussion is understood to apply to these other types of sensors, monitoring units and/or physiological information.

The sensor 114 may be situated at one or more portions of the MCTU and may sense related parameters, form corresponding sensor information, and provide this sensor information to the controller 120 for further processing. For example, the sensor 114 may include one or more sensors such as cardiac sensors configured to sense electrical signals at the skin of the patient. In this way, the sensor 114 may sense analog cardiac signals, may form corresponding sensor information (e.g., cardiac signals (ECG), etc.) and provide this information to the controller 120 for further analysis and/or processing. The sensors 114 may be further distributed throughout the system, such as at the MS, etc. The controller 120 may control the overall operation of the MCTU 102. Although three sensors 114 are shown for illustration, there may be more or less of these sensors. In some embodiments, the sensors 114 may be coupled to the controller 120 via leads 115 that may be deposited upon the substrate 110 or may be formed using separate wires. In accordance with embodiments, one or more of the sensors 114 may be wirelessly coupled to the controller 120 (e.g., via Bluetooth^{™}). The sensors 114 may be formed integrally with, or separately from, the substrate 110 and may be distributed in a desired pattern relative to the substrate 110 to collect cardiac information from the skin of the patient.

The substrate 110 may include a flexible substrate that may have an adhesive 124 configured to releasably couple to the skin of the patient. The adhesive 124 may form a layer that may be located on at least one side of the substrate 110 in a continuous or non-continuous manner. The adhesive 124 may include an electrically conductive adhesive layer configured to pass electrical signals from the skin of the patient to the sensor 114 and may be medical grade. The substrate 110 may have any desired shape and size suitable for its use and it is envisioned that it may be continuous or discontinuous (e.g., formed from one or more sections that may be coupled together). The substrate 110 may be formed from any suitable material or materials such as a medical grade polymer that may include one or more layers superimposed upon each other. The substrate 110 may include instructions printed thereon for instructing a user on proper, placement, orientation, and use.

In some embodiments, one or more of the controller 120, the Tx/Rx 122, the memory 126, and the sensor 118 (e.g., one or more), may form at least a portion of a control unit (CU) 119 that may be removably coupled to the substrate 110 via a coupler that may or may not further couple one or more of the sensors 114 to the CU 119. Accordingly, the substrate 110 including the sensors 114, may be changeable for the convenience of the user, while the CU 119 may be removable from the substrate 110 (and sensors 114) and may be reused with a new substrate 110.

The controller 120 may be configured to communicate with the MS 104 through the TX/RX 122 using any suitable communication method or methods such as via a Bluetooth^{™} connection or the like and may synchronize an internal clock with a clock of the MS 104. The controller 120 may obtain the SSI from the MS 104 and store this information in the memory 126 for reference and/or use at a later time and/or to configure its operation. During operation, the controller 120 may receive analog signals from the sensor 114, amplify and/or condition these analog signals, convert these signals (e.g., using an analog-to-digital (A/D) converter) to digital signals (e.g., at a desired sampling rate (as may be set in the SSI)) to form physiological signals, such as electrocardiogram signals, which may then be formed into physiological information, such as the ECGI. Further, the controller 120 may process the ECGI, for example in accordance with the SSI, to determine whether any abnormalities, such as arrhythmias, irregular heart rhythm, high blood pressure, SpO2 levels, etc., are present. When it is determined that an abnormality is present, the controller 120 may obtain the current time from its clock and form a corresponding time stamp (TS) (e.g., corresponding to a current time or other time when the ECG was captured) and transmit an activate voice recording mode command (AVRM) with the TS (e.g., as TS-AVRM) to the MS 104. The controller 120 may also obtain an ECGI which may have a duration in time (e.g., 90 seconds as may be set forth in the SSI, although other durations are contemplated) and may time-stamp the ECGI in accordance with the TS to form the TS-ECGI.

Thus, the MCTU 102, the MS 104 and/or the CSC 106 may detect abnormalities and/or other condition(s) by processing the physiological information, such as the ECGI. When an abnormality and/or other condition(s) is detected, the MS 104 may, for example, start a two-way voice dialogue with the patient (e.g., between the MS 104 and the patient) to obtain information related to the context of the detected abnormality (e.g., "What were you doing?"; "Did you experience any symptoms?"; "Are you OK now?"; etc.). In some embodiments, the controller 120 of the MCTU 102 may receive a signal, such as a TS, TS-ECGI, etc., from the MS 104, indicating a request for capturing/forming/transmission of an ECGI or a TS-ECGI from to the MS 104. For example, this may occur when the MS 104 is activated, such as through a gesture from the patient, to indicate that the patient is feeling a symptom.

The sensor 118 may include one or more sensors (such as a button) which, when a touched, depressed, etc., by the patient, may transmit a corresponding signal to the controller 120. The controller 120 may then determine that a touch sensor (of the sensors 118) has been touched and may in response obtain a corresponding time (e.g., of the corresponding detected touch) from its clock and form a corresponding TS corresponding to the touch. Thus, for the sake of clarity, it will be assumed that the TS may substantially correspond with the detected touch although a determined timing offset is also contemplated. The controller 120 may capture a corresponding ECGI and may then form information including an activate voice recording mode command (AVRM) and include this information with the TS (e.g., TS-AVRM) information and transmit this information (e.g., via the Tx/Rx 122) to the MS 104 (e.g., via a Bluetooth^{™}) for further processing. Upon receiving the TS-AVRM, ECGI (or TS-ECGI), the MS 104 may activate the voice recording mode (VRM) as discussed herein. In accordance with embodiments of the present system, the MCTU 102 may send the ECGI or the TS-ECGI to the CSC 106, directly or otherwise through an intermediary device, and send the TS-AVRM to the MS 104 to activate the VRM so that symptom and/or context information may be formed (e.g., the URI).

In accordance with embodiments of the present system, when the controller 120 determines that a touch sensor (of the one or more sensors 118) has been touched, the controller 120 may obtain an ECGI and a TS corresponding to when the touch sensor is touched. Thus, for example, the controller 120 may obtain the ECGI starting at the time indicated by the TS and having a given duration. However, in some embodiments, the ECGI may start at a certain time before the TS, in embodiments when available, such as 10 seconds etc. before the touch, as may be set forth in the SSI. In accordance with embodiments of the present system, the controller 120 may buffer one or more of the ECGI and the TS in the memory 126 for later transmission.

In accordance with embodiments of the present system, wherein all recorded physiological information (e.g., the ECGI) is stored and/or forwarded to the MS 104 and/or the CSC 106, the TS may be synchronized and/or otherwise associated with the corresponding information portion to facilitate retrieval, storing, association, etc., with the URI. Further, should one or more parts of the system including the MCTU 102, the MS 104 and the CSC 106 be within a different time zone and/or be moved to a different time zone from another part of the system (such as when the patient is traveling or daylight savings is begun or ended), the logging of information including the physiological information, symptoms, context, etc., may be logged in a single time reference, such as in UTC-time, to facilitate the association of the different information together.

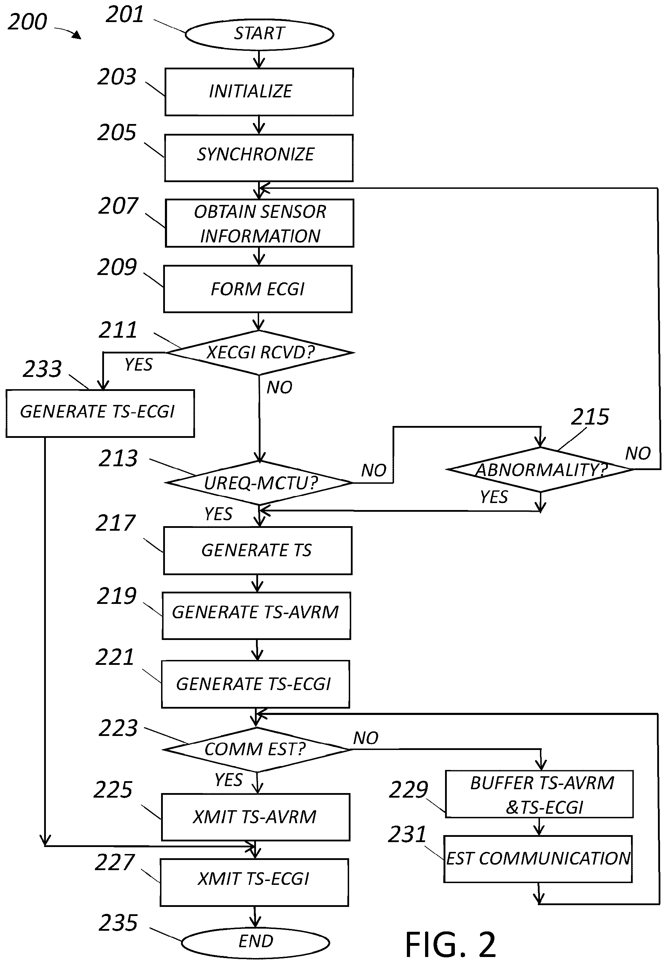

An operational process performed by the MCTU communicating with a MS of the patient will now be described with reference to FIG. 2 which shows an illustrative functional flow diagram performed by a process 200 in accordance with embodiments of the present system. The process 200 may be performed using one or more processors, computers, controllers, etc., communicating over a network and may obtain information from, and/or store information to one or more memories which may be local and/or remote from each other. The process 200 may include one of more of the following acts. In accordance with embodiments of the present system, the acts of process 200 may be performed using a controller operating in accordance with embodiments of the present system, such as the controller 120 of the MCTU. Further, one or more of these acts may be combined and/or separated into sub-acts, may be performed serially and/or in parallel. Further, one or more of these acts may be skipped depending upon settings, embodiments, etc. For the sake of clarity, the process may be described with reference to a single MCTU and MS. However, without limitation, it should be understood that the process may employ a plurality of MCTUs, MSs, etc., each of which may include a separate workflow such as a sub-workflow. In operation, the process 200 may start during act 201 and then proceed to act 203.

During act 203, an initialization may be performed during an initialization routine. During initialization, the MCTU may load SSI which may include operating parameters such as, instructions and settings for the initialization routine, sampling rates, system settings (e.g., types of sensors and their parameters, etc.), patient ID, and identification information of the MS of the patient (e.g., an MS ID) to establish communication between the MCTU and the MS (e.g., via any suitable connection method such as a Bluetooth^{™}, etc.). The SSI may be stored locally in the memory of the MCTU and/or in another memory of the system such as in the memory of the MS. After loading the SSI, the controller may set system settings accordingly. During the initialization, the MCTU may attempt to establish a communication link (e.g., a Bluetooth^{™}, etc.) with the MS (e.g., an MS of the patient, hereinafter the MS). However, for example, when a link is not established between the MCTU and the MS, the MCTU may obtain the SSI from its own memory and operate independently until communication with the MS is established. In some embodiments, the SSI may set forth one or more times and/or a rate (e.g., every 15 minutes) of acquiring an ECGI which when coupled to the MS, is pushed (e.g., transmitted) to the MS without requiring a request by the patient. After completing act 203, the process may continue to act 205.

During act 205, if communication is established with the MS, a clock of the MCTU may be synchronized with the clock of the MS. Synchronization is utilized to assure that the timestamps transmitted by the MCTU and/or MS refer to the same time. Synchronization may be performed, for example, by sending a timestamp (e.g., from MS to MCTU), provided there is neglectable or a known transmission time. In accordance with embodiments, synchronization may be performed, for example, by using one or more sophisticated protocols, like those based on Network Time Protocol (NTP) or by synchronization of each to another (common) network timing. It is envisioned that the synchronization routine may be run during operation as may be set forth in the SSI (e.g., every two hours, every two days, etc.) to resynch the clock of the MCTU with the clock of the MS. As previously discussed, the synchronization may be to a single common time reference, such as UTC-time. After completing act 205, the process may continue to act 207.

During act 207, the MCTU may obtain sensor information from one or more sensors. Accordingly, the controller of the MCTU may monitor and obtain physiological information, such as cardiac signal information, from the cardiac sensors (e.g., see one or more sensors 114 of FIG. 1). The cardiac signal information may include analog cardiac signals which may be amplified by amplifiers and/or conditioned by signal conditioners of the MCTU for further processing such as for forming the ECGI in accordance with embodiments of the present system. The controller may also monitor other sensors of the system, such as a touch sensor 118, for signals indicative of a touch or pattern of touches (e.g., a double-tap) which may be indicative of a patient request (UREQ) initiated on the MCTU as may be set in the SSI. For example, in the present embodiments, the SSI may set the user indication that a symptom is being felt by the patient to a double tap.

When it is determined that the touch sensor has, for example, been double tapped by the patient, the controller may determine to generate the user request (UREQ). Signals or patterns mapped to the UREQ (e.g., that are utilized to generate the UREQ) may be stored in the SSI and may include any suitable touch(es), gesture(s), patterns, and/or interaction with key(s), button(s), etc. However, in yet other embodiments, other types of keys and/or patterns may be mapped to the UREQ and may be stored in a memory of the system such as in the SSI for later use. It is envisioned that the system may generate an interface (e.g., an audio interface, a graphical user interface (GUI), etc.) with which the patient may interact via the UI to select and/or change the SSI settings.

Accordingly, a user may interact with the rendered interface via the UI to select the UREQ to be mapped to one or more keys, dedicated buttons, gestures, patterns, etc. For example, the UREQ may be mapped to one or more taps (e.g., a double tap in the present embodiments), a pattern (e.g., an X pattern input via a touch sensor such as a touch screen), and/or touching, pushing, etc., a dedicated button or key, etc. In accordance with embodiments of the present system, the interaction for generating the UREQ may be set to a default interaction by the SSI without requiring selection by the patient. Furthermore, an interaction may operate directly for initiating operation in accordance with embodiments of the present system without being mapped to the UREQ. For example, the interaction with the MCTU may cause directly (e.g., be electrically wired to) the capture of physiological data, the forming and transmittal of the physiological information and the TS, and the receipt of which by the MS, may initiate the VRM and subsequent forming of the URI.

Further, the MCTU may monitor communication with the MS to receive information, such as the TS and corresponding information such as a transmit ECGI request (XmitECGI) which may be transmitted from the MS to the MCTU, for example, along with the TS such as TS-XmitECGI. The XmitECGI may include a command for the MCTU to capture and/or transmit the ECGI. In some embodiments, the XmitECGI may be generated in response to a patient performing a particular interaction with the MS, such as depressing a dedicated button, through a gesture (e.g., a chopping motion of the MS, a double tap through a UI of the MS, etc.), etc. After completing act 207, the process may continue to act 209.

During act 209, the MCTU may form the ECGI based upon processed analog cardiac signals. For example, the controller 120 may process the acquired (analog) cardiac signals and form corresponding ECGI which may, then be stored locally in the memory of the MCTU for further processing and/or subsequent transmittal to the MS in accordance with system settings as may be set forth in the SSI. In accordance with embodiments, the ECGI may be formed in response to a user interaction, in response to a UREQ, in response to a received XmitECGI from the MS, periodically and/or at set times as described herein. After completing act 209, the process may continue to act 211. During act 211, the process may determine whether the TS-XmitECGI was received. Accordingly, when it is determined that the TS-XmitECGI was received, the process may continue to act 233. However, when it is determined that the TS-XmitECGI was not received, the process may continue to act 213.

During act 213, it may be determined whether the UREQ was generated. When it is determined that the UREQ was generated, the process may continue to act 217. However, when it is determined that the UREQ was not generated, the process may continue to act 215. During act 215, the controller may determine whether an abnormality has occurred. In accordance with embodiments of the present system, the ECGI may be analyzed to determine whether one or more abnormalities such as an arrhythmia, etc., are present in the ECGI. For example, the controller may run one or more applications which may analyze the ECGI to determine whether there is an irregular heartbeat indicated by the ECGI. In the event that an abnormality is detected in the ECGI, the process may store an indication that an abnormality has been detected together with (e.g., associated with) the ECGI and thereafter, the process may continue to act 217. However, when it is determined that an abnormality has not occurred, the process may return to act 207, wherein a further ECGI may be formed, etc. In accordance with embodiments of the present system, the abnormality may be detected during a different portion of the process, such as during act 209, after forming the ECGI.

During act 217, the process may generate a TS corresponding substantially to the time that the UREQ was generated (e.g., the time of the user's interaction with the MCTU that generates the UREQ) and/or the time the ECGI was formed. As readily appreciated, with the speed of processing, the time that the UREQ was generated and the time the ECGI was formed may be substantially the same time. In any event, only one TS may need to be generated. In operation, the process may obtain the time from a clock of the MCTU. After completing act 217, the process may continue to act 219 during which, the controller may generate information such as a time-stamped AVRM (TS-AVRM) which includes an AVRM command associated with a TS that, for example, corresponds to the time of the UREQ. In accordance with embodiments, a TS-UREQ may be sent in place of the TS-AVRM to subsequently set the VRM of the MS when received. In these embodiments, the TS-AVRM may not be needed (e.g., step 219 may be skipped). After completing act 219, the process may continue to act 221.

During act 221, the MCTU may generate a TS-ECGI using the ECGI which corresponds, in time, with the time stamp as set forth in the TS. For example, a current ECGI or a ECGI stored in memory may be retrieved. The ECGI when stored in the memory may include information related to the time that it was formed. Accordingly, the controller may retrieve the ECGI that is associated with the time of the TS. After completing act 221, the process may continue to act 223 during which the controller may determine whether communication between the MS of the patient (e.g., a registered MS) is established (COMM EST).

When it is determined that communication with the MS is established, the process may continue to act 225. However, when it is determined that communication with the MS is not established, the process may continue to act 229. In accordance with embodiments of the present system, communication between the MCTU and the MS is established using a wireless communication link, such as a short-range wireless communication (e.g., Bluetooth^{™}) or the like. During act 225, the controller, for example through operation of the TX/RX (e.g., TX/RX 122), may transmit the TS-AVRM to the MS of the patient. Accordingly, the process may communicate with the MS using, for example, the established communication link. After completing act 225, the process may continue to act 227 during which the controller may transmit the TS-ECGI to the MS. As readily appreciated, the AVRM, the TS and the corresponding (associated) ECGI may be transmitted together. After completing act 227, the process may continue to act 235 where it may end. For example, should a cardiac monitoring period for the MCTU end, such as following a week of cardiac monitoring, then the process would continue to act 235 where it may end. While only one transmission of the TS-ECGI is illustratively described, it is readily appreciated that following act 227, in place of continuing to act 235 and ending, the process may continue to act 207 to continue monitoring as described.

During act 229 (i.e., when there is no established communication link between the MCTU and the MS), the MCTU may store the TS-AVRM and the TS-ECGI in a memory of the system, such as in the local memory 126, for later processing, such as for transmission when a communication link is established between the MCTU and the MS. In accordance with embodiments of the present system, the storage of the TS-AVRM and/or the TS-ECGI in a memory (e.g., such as a list of any TS, AVRM, ECGI that has not been transmitted to the MS for association with symptom/context information (e.g., corresponding URI)) assures that symptoms and/or context for each of the TS-ECGI, wherein an irregularity is indicated by the patient and/or is detected by the system, may be obtained from the user, even when communication is not available between the MCTU and the MS.

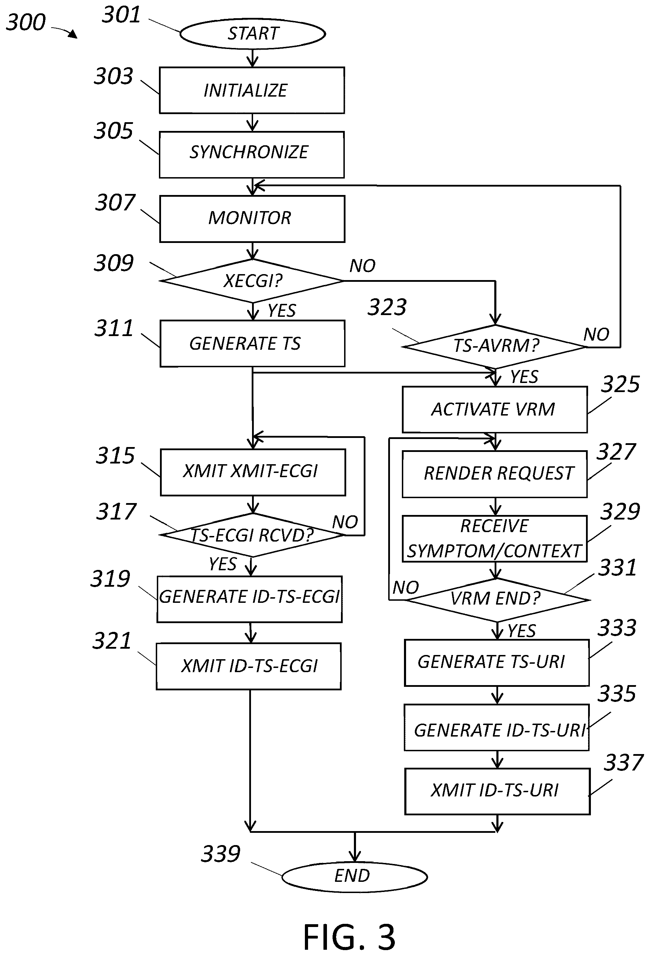

When communication is not available between the MCTU and the MS, the patient may be asked to provide symptoms and/or context for each of the TS-ECGI after the communication is reestablished. Accordingly, medical data, associated events, symptoms and/or context may be provided to the CSC and/or clinician rather than being absent as may occur with prior systems. Thus, if the MCTU is not able to communicate with the MS (e.g., for any reason such as because the MS is out of communication range, the MS battery is depleted, etc.), the MCTU may be configured to buffer or otherwise store the TS-ECGI and the associated ECGI in a memory until communication with the MS is established. Thus, if a patient experiences symptoms at 9:10, 10:05, and 11:00 am, the system will generate corresponding requests for the MCTU to form corresponding time stamps, form ECGI associated with these time stamps (e.g., TS-ECGI), and store the TS-ECGI in the memory of the MCTU along with a corresponding AVRM until communication with the MS is established or reestablished (e.g., at 1:00 pm). Thereafter, when communication between the MCTU and the MS is established, the TS-ECGI and the AVRM may be transmitted to the MS. In response, the MS may activate the VRM to render information, such as a voice dialog, etc., to interact with the patient such that the patient may provide symptoms and/or context to the previously undescribed ECGI (e.g., which were captured/stored at 9:10, 10:05, and 11:00 am) one-by-one. Accordingly, the MS may be configured to inform the user of the TS of the ECGI and thereafter obtain a response from the patient to form corresponding TS-URI (e.g., see FIG. 3).

After completing act 229, the process may continue to act 231 during which an attempt is made to establish communication between the MCTU and the MS. For example, status information may be rendered to the patient on a rendering device such as a speaker of the system. In accordance with embodiments of the present system, the status information may inform the patient that the MCTU may be out of range of the MS and/or otherwise cannot communicate with the MS. Accordingly, in response the patient may try positioning the MS within communication range of the MCTU or may set/reset settings (e.g., turn off airplane mode, turn on Bluetooth^{™} communication, etc.) such that communication between the MCTU and the MS may be established or resumed. As readily appreciated, the MCTU may periodically attempt to establish communication with the MS without informing the user. After completing act 231, the process may continue to act 223 and thereafter proceed as previously described.

During act 233 (i.e., after it is determined that the TS-XmitECGI was received during act 211), the controller may generate TS-ECGI using the ECGI which corresponds in time to the TS of the TS-XmitECGI. This act may be similar to act 221, accordingly, reference may be made to act 221 for a further description. During the present act, the process may obtain a currently occurring ECGI or may obtain an ECGI from a memory of the system that corresponds to the TS of received TS-XmitECGI. After completing act 233, the process may continue to act 227 and continue as previously described.

As discussed herein, one or more MS of the user may perform an operational process in accordance with embodiments of the present system. An illustrative operational process performed by the one or more MS of the patient is described with reference to FIG. 3 which shows an illustrative functional flow diagram performed by a process 300 in accordance with embodiments of the present system. The process 300 may be performed using one or more processors, computers, controllers, etc., communicating over a network and may obtain information from, and/or store information to one or more memories which may be local and/or remote from each other. The process 300 may include one of more of the following acts. In accordance with embodiments of the present system, the acts of process 300 may be performed using a controller (e.g., controller 190) operating in accordance with embodiments of the present system. Further, one or more of these acts may be combined and/or separated into sub-acts. In addition, one or more of these acts may be skipped depending upon settings, embodiment, etc. For the sake of clarity, the process may be described with reference to a single MS unless the context indicates otherwise. However, without limitation, it should be understood that the process 300 may employ a plurality of MS, each of which may include a separate workflow such as a sub-workflow. In operation, the process 300 may start during act 301 and then proceed to act 303.

During act 303, the process may be initialized by performing an initialization routine. During initialization, the MS may load SSI which may include operating parameters such as, instructions and settings for the initialization routine, sampling rates, system settings (e.g., types of sensors and their parameters, etc.), patient identification information (ID), and identification information of the MCTU of the patient (e.g., a MCTU ID) to establish communication with the MCTU (e.g., via any suitable connection such as a Bluetooth^{™}, etc.). The SSI may be stored locally in the memory of the MS or in any another memory of the system such as in in the cloud, etc. After loading the SSI, the controller of the MS may set system settings accordingly.

During the initialization, the MS may attempt to establish a communication link (e.g., a Bluetooth^{™}, etc.) with the MCTU of the patient. The MS may also attempt to establish a communication link with the CSC and/or with another MS of the system. In some embodiments, upon establishing a communication link with the MCTU, the MS may transmit the SSI to the MCTU and/or another MS so that most recent and/or corresponding SSI may be used by both the MCTU and the MS(s). It should be appreciated that if a communication link between the MS and the MCTU may not be established, or is lost, either or both the MCTU and the MS may function independently, or substantially independently, of each other in accordance with embodiments of the present system until a communication link is established or reestablished.

After completing act 303, the process may continue to act 305 when communication is established with the MCTU so that a clock of the MCTU may be synchronized with the clock of the MS. Accordingly, the MCTU and the MS may perform a clock synchronization routine to ensure that the timestamps (TS) transmitted/utilized by the MCTU and/or the MS utilize the same time reference. As readily appreciated, the time synchronization may also occur through each of the MCTU and the MS synchronizing to a common third device/time reference and/or through another process as discussed herein. After completing act 305, the process may continue to act 307.

During act 307, the process may monitor one or more sensors of the MS or a connected (other) MS, smart watch, smart ring, smart headband, Healthdot^{™}, sensor patch, etc. (each generally referred to herein as token) or the like to obtain information from these one or more sensors that indicates that the patient is feeling a symptom or otherwise wants an ECGI to be captured by the MCTU. As discussed with reference to FIG. 2, generation of the XmitECGI (the request from an MS to the MCTU for the MCTU to capture a ECGI) may be mapped to one or more keys, buttons, touch-screen input patterns, gestures (touch and/or touchless) such that upon detection of one or more of these keys, buttons, touch-screen input patterns, gestures, etc., the MS may generate the XmitECGI and may thereafter, access a clock of the system to obtain a current time to generate the TS as discussed with reference to act 311. For example, a chopping motion (gesture) performed by the patient on the MS may be utilized to generate the XmitECGI. Accordingly, when it is detected that the MS is being moved in a chopping motion, the MS may generate a corresponding XmitECGI.

In embodiments of the present system, when the patient moves the MS in a specific way that is mapped to a XmitECGI, such as shaking or flipping the MS upside down, twisting and/or shaking a ring-shaped MS a number of times, etc., the MS upon detecting this action, may generate a corresponding XmitECGI and subsequently activate the VRM (e.g., see act 325). Accordingly, the MS may monitor sensor information from one or more of its sensors (e.g., see, FIG. 1, the sensor(s) 195), such as from touch sensors (e.g., a touch-screen), a keypad, gesture sensors (e.g., one or more accelerometers, gyroscope, magnetometers, proximity sensors, optical sensors, lidar, gravity sensors, etc.), and process this information to determine whether the information corresponds with a command mapped to the XmitECGI.

It is envisioned that the MS may generate and render a user interface with which the patient may interact to map one or more one or more keys, input strokes, voice commands, and/or gestures to the XmitECGI and/or it may be mapped to a default action. When more than one MS is being employed by the patient, any MS that generates the XmitECGI may transmit this information to another MS for forwarding to the MCTU or may forward the XmitECGI directly to the MCTU.

During the monitoring (e.g., during act 307), the MS may also monitor for information received from the MCTU such as ECGI, TS, TS-AVRM, TS-ECGI, other information, and/or combinations thereof. The process may further monitor for information transmitted from other MS such as a smartwatch, token, etc., associated with the patient. The received information may then be processed in accordance with embodiments of the present system. Monitoring for this incoming information may continue during many of the other acts described herein. After completing act 307, the process may continue to act 309 wherein it is determined whether an XmitECGI was generated or received (e.g., from another MS of the system). Accordingly, when it is determined that the XmitECGI was generated or received, the process may continue to act 311. However, when it is determined that the XmitECGI was not generated or received, the process may continue to act 323.

During act 323, the process may determine whether a TS-AVRM (signal) was received (e.g., from the MCTU). Accordingly, when it is determined that the TS-AVRM was received, the process may continue to act 325. However, when it is determined that the TS-AVRM was not received, the process may repeat act 307 wherein the monitoring of information is continued as previously described.

During act 311 (i.e., after it is determined that the XmitECGI was generated or received during act 309), the process may generate TS corresponding to the time the XmitECGI was generated. As readily appreciated, in an event wherein the XmitECGI was received from another MS, an associated TS may already be provided that is associated with the XmitECGI and as such, act 311 may be skipped. After completing act 311, the process may continue to act 315 and act 325. It should be appreciated that the process may run acts 315 through 321 and 325 through 337 serially or in parallel with each other.

During act 315, the MS may transmit the XmitECGI with the corresponding TS to the MCTU using any suitable connection such as a wireless connection (e.g., Bluetooth^{™}). After completing act 315, the process may continue to act 317, wherein the MCTU may determine whether the TS-ECGI was received. Accordingly, when it is determined that the TS-ECGI was received, the process may continue to act 319. However, when it is determined that the TS-ECGI was not received, the process may repeat act 315.

During act 319, the MCTU (e.g., the controller of the MCTU) may generate information such as ID-TS-ECGI which is the TS-ECGI with the corresponding (patient) ID. Accordingly, the current TS-ECGI may be obtained (formed) and may be associated with the corresponding (patient) ID to form the ID-TS-ECGI which may be time-stamped ECG information that is associated with the ID. In other embodiments, association with the ID may occur at the MS and/or the CSC. After completing act 319, the process may continue to act 321 during which, the MCTU may transmit the ID-TS-ECGI to the MS and/or CSC. Accordingly, the process may perform this transmission using any suitable transmission method (e.g., a 5G wireless cellular, etc., see Network 108, FIG. 1). After completing act 321, the process may continue to act 339 where the process may end. While only one transmission of the ID-TS-ECGI is illustratively described, it is readily appreciated that following act 321, in place of continuing to act 339 and ending, the process may continue to act 307 to continue monitoring as described.

A VRM will now be described with reference to act 325 through act 331 which may be performed during a VRM (mode) of the MS. It should be noted that although act 325 is illustratively shown to follow from other acts, it may also be performed, for example, immediately following act 307 after an XmitECGI action by the patient is detected by the MS and before the ECGI is received from the MCTU. In any event, during act 325 (e.g., it is determined that a TS-AVRM (signal) was received during act 323), a voice recording mode (VRM) may be activated, such as at a MS that received the AVRM Accordingly, the process may activate a voice recording application (app) which may obtain voice request information (VRI) from a memory of the system. In some embodiments, the VRI may be stored in the SSI and may be set and/or reset by the system, clinician, and/or patient. The VRI may include content, textual, graphic, and/or audio information. For example, the VRI may include request information having a format ["message", [time]], where the time may correspond with the corresponding time stamp as indicated in the TS. For example, assuming the TS is 9:10 pm, the VRI may cause a message such as "Please describe your symptom of [9:10 pm]" to be rendered. Similarly, if the TS is 10:01 am, the rendered message may be "Please describe your symptom of [10:01 am]." The process may do this for all corresponding TSs. After completing act 325, the process may continue to act 327.

During act 327, the process may cause to be rendered a request on a rendering device of the system to inform the patient to "Please describe your symptom of 9:10 pm." This request may be rendered as set forth in the VRI in accordance with the TS. It is envisioned that the VRI may further include information which may indicate selected rendering devices such as a speaker and/or haptic device in accordance with embodiments of the present system. For example, the speaker may be a speaker of the MS or may be a speaker coupled to the MS via any suitable connection such as a Bluetooth^{™} connection. This may facilitate a patient to provide symptoms while mobile such as when driving a car, etc.

In accordance with embodiments of the present system, the MS may activate the voice recording mode (VRM), where a voice dialog with the patient may be performed. For example, when the voice recording mode on the MS is activated, the controller may control a corresponding UI to render information (as may be stored in the SSI and/or the VRI) to inform the patient about activation of the VRM. For example, information may be rendered (e.g., audibly) such as "voice recording activated" or "please describe your symptom," on a rendering device of the system, such as a speaker associated with (e.g., part of) the MS. Haptic feedback may also be generated to draw the attention of the patient such as by causing the MS to vibrate, etc. The speaker may be a speaker coupled to the MS (e.g., a headset, a Bluetooth enabled speaker such as a car speaker, etc.).

After completing act 327, the process may continue to act 329 wherein the process may await a response from the patient. It is also envisioned that the process may provide the patient with a list of symptoms for the convenience of the patient. In embodiments, the system may provide the list of (possible) symptoms if requested by the patient. Accordingly, the process may interact with the patient using an interactive voice menu system. For example, if a response from the patient is not received in a certain time (e.g., 10 seconds, although other times are also envisioned), the process may render a list of symptoms on the rendering device (e.g., the speaker) for the convenience of the patient. Thereafter, the process may await a response. Upon receiving a symptom and/or context description (e.g., a description of one or more symptoms/context from the patient), the process may determine that the symptom/context description is completed for a corresponding TS and may render information indicating such (e.g., "Symptom description completed") on the rendering device of the MS. The system may then process the user input symptom/context description to form user (symptom) response information (URI) in association with the corresponding TS (e.g., see act 333).

To collect the patient responses (e.g., symptoms, context, stop recording command, etc.), the MS may monitor sensors, such as a microphone, for one or more responses from the patient. For example, the patient may speak to describe the symptom, symptoms and/or context. Thereafter, when finished, the patient may speak or utter a dedicated word or words such as "Stop recording" to end the receipt of the patient response as determined during act 331. In accordance with embodiments of the present system, the patient may touch a sensor of the MCTU and/or on the MS in the same or a similar manner as was used to form the previous user request (e.g., to activate the VRM). Further, the MCTU and/or on the MS may have a different touch, gesture, etc., to stop the VRM than was used to initiate it. In any event, the MCTU and/or the MS may generate a stop recording command.

When the stop recording command is initiated at the MCTU or another MS (an MS not in the VRM), the MCTU or other MS may transmit this command to the MS in the VRM which in response, may then determine to stop recording (e.g., see act 331). Other methods to stop recording are also envisioned and may be mapped to a stop recording command and stored in the SSI for later use. It is envisioned that the process may employ a voice-to-text and/or AI to interact with the patient and/or determine what was uttered by the patient (e.g., symptom description, context, and/or stop recording command). In some embodiments, the app may ask "shall I stop recording?" when it has not heard the patient speaking for a time period such as Y seconds, where Y may be set by the system and/or user and stored in the SSI. Then, when it is determined (e.g., by the process) that the patient has uttered "Yes" the process may stop recording. However, when it is determined that the patient has uttered "No" or some other indication that the VRM should not be ended, the process may continue to record the patient responses to form the URI. In some embodiments, if it is determined that the patient did not speak anything or no words were detected during act 329, the process may repeat act 327 a number of times (e.g., P times, where P is an integer that may be set and/or reset by the system and/or user and stored in the SSI).

In addition, the MS may repeat the process (e.g., acts 325 to 331) to collect a patient response for each TS-AVRM and/or XmitECGI generated and/or received. Further, if communication between the MCTU and the MS is interrupted, the process may store information for later use when communication is reestablished. Accordingly, a further benefit of the present system is that if communication is interrupted, for example, between the transmission of the TS-XmitECGI by the MS to the MCTU and the receipt by the MS of the ECGI, the MCTU may capture and form the ECGI at the desired time (e.g., as indicated by the received TS). Thereafter, after communication between the MCTU and the MS is reestablished, the MCTU may transmit the ECGI, and the MS may form corresponding URI at a later time such that ECGI as well as associated symptoms and/or context are not absent when the patient's information is evaluated by the clinician.

When a symptom is indicated by the patient and/or detected by the MCTU but the MCTU is not in communication with the MS, the MCTU may maintain a list of undescribed symptom events in its memory. In this way, when the patient indicates a symptom is being felt and/or an irregular ECG is detected, the MCTU may form an ECGI. In the event that the MCTU cannot contact the MS, the MCTU may add the symptom event (e.g., the ECGI with the timestamp) to this list. Later, when the connection between the MCTU and MS is restored, the undescribed symptom events (e.g., the ECGI and the associated TS) are sent to the MS and rendered to the patient for manual annotation or voice recording so that the MS may form the URI as described. The patient may be informed and reminded about TS-ECGI that has not been associated with URI by means of a sound and/or vibration, display, etc. Accordingly, the present system may greatly aid in the diagnosis and treatment of the patient since symptom descriptions and/or context are not lost or otherwise absent from the patient information that is evaluated as in prior systems. In this way, the present system addresses problems not solved by the prior systems and thereby greatly improves over the prior systems and methods.

Although sample interactions with the patient are illustratively described, other interactions with the patient are also envisioned as may be set forth by the system (e.g., default UVI), clinician, and/or user. It is also envisioned that artificial intelligence (AI) engines may be employed to interact with the patient and provide interaction with the patient which may include an interactive voice menu.

After completing act 329, the process may continue to act 331 during which, it is determined whether to end the VRM. Accordingly, when it is determined to end the VRM, the process may continue to act 333. However, when it is not determined to end the VRM, the process may repeat act 327 and/or act 329. The process may determine to end the VRM when it is determined that the symptom/context description has been completed for the TS. Accordingly, when the symptom description has not been completed for the TS, the process may repeat act 327 and/or act 329. In some embodiments, the process may perform the VRM for each TS.

During act 333 (i.e., after recording of the patient response is completed), information may be rendered on a rendering device, such as a speaker associated with the MS, to inform the patient that the symptom description has been completed such as an audibly rendered "symptom description completed" or the like. In addition, a controller may process the symptom, symptoms, context, etc., uttered by the patient and form corresponding URI which may be time stamped by the TS to form TS-URI. After completing act 333, the process may continue to act 335 wherein the MS may generate ID-TS-URI which is the TS-URI associated with the corresponding patient ID information. Accordingly, the process may obtain the current TS-URI and associate a corresponding patient ID with it to form corresponding ID-TS-URI which may be time-stamped URI with the corresponding patient ID.

As discussed herein, the ID may be associated with the URI at another time and/or by another system (e.g., by the CSC). After completing act 335, the process may continue to act 337 wherein the MS may transmit the ID-TS-URI to the CSC. Accordingly, the process may perform this transmission using any suitable transmission protocol (e.g., 5G wireless cellular, etc.) via the network (e.g., see network 108, FIG. 1). After completing act 337, the process may continue to act 339 where the process may end. While only one VRM is illustratively described, it is readily appreciated that following act 337, in place of continuing to act 339 and ending, the process may continue to act 307 to continue monitoring as described.

In some embodiments, the MCTU may transmit portions of the ECGI such as ECG strips (e.g., 90 second strips, etc. about the TS) to the MS which may conserve system resources. However, it is also envisioned that in some embodiments the MCTU may transmit all ECG data (e.g., ECGI) to the MS at periodic or non-periodic time intervals as may be set by the system, clinician, and/or patient and stored in the SSI. For example, in these embodiments it is envisioned that the MCTU may transmit ECGI to the MS every five minutes, etc., which data may include ECGI of the last 5 minutes that is collected by the MCTU. When the patient informs the MCTU about the occurrence of a symptom (e.g., by performing an action that generated a UREQ), this information may be conveyed to the MS by transmitting a TS-AVRM signal to the MS which, in response, may generate an ECG strip from the ECGI that it has previously received from the MCTU and may activate a VRM to start a voice dialogue with the patient to describe the symptom as discussed herein.

Accordingly, embodiments of the present system may automatically activate a voice recording mode of an application operating in accordance with embodiments of the present system, for example, when the patient uses touch (e.g., tapping a sensor, a screen and/or button of a phone, a smart watch, etc.) and/or gestures (e.g., shaking a phone, flipping the phone, making a chopping gesture with a smart watch, twisting a smart ring around a finger a number of times, etc.) to inform the system that a symptom (e.g., an event) has been experienced. Once the system is informed, it may obtain information such as a time stamp of the symptom, a corresponding ECGI, add patient ID information to this information and forward this information to a CSC for further analysis as described in more detail herein.

Although a single CSC, MS, and MCTU are shown, it is envisioned that embodiments of the present system may be operative with a plurality of any of the CSC, MS, and/or MCTU. For example, the system may include a plurality of MCTUs, one or more for each patient of a plurality of patients. Similarly, a plurality of CSCs may be employed and may be selected as desired, such as by geographic location of the MS of the patient, etc. In addition, a plurality of MS may be employed by a patient. For example, a first MS may be a smartphone associated with the patient while a second MS may be a smart watch, ring, or other wearable (referred to generally as a token), etc. In these embodiments, the symptom recording process may also be initiated by a third device (e.g., the second MS) that is connected to the MCTU and/or the first MS, such as a watch or other wearable (referred to as token). In these embodiments, the patient may utilize the token to indicate the occurrence of a symptom. As described, the symptom recording process may also be initiated and executed by the second MS that is a smartwatch (e.g., with microphone and speaker) that is connected to the MCTU (e.g., a heart monitor) and/or the first MS (e.g., a smart phone). In this embodiment, the smartwatch may be utilized to record the symptom and form the URI, which it may subsequently send to the first MS and/or the CSC. It should be noted that the second MS (e.g., token, smartwatch, etc.) may not be directly coupled to the MCTU but may be coupled via the first MS (e.g., a smart phone) instead. Accordingly, the signals from the second MS (e.g., token/smartwatch) to the MCTU (e.g., cardiac sensor) may go via the first MS (e.g., smart phone).

It is also envisioned that if an emergency is detected, a geographic location of the MS and/or MCTU may be determined and emergency services may be informed, in accordance with system and/or user settings. It should also be appreciated that materials employed by the MCTU 102 and/or portions thereof, may include medical grade materials suitable for their use.

By operating using touchless requests and employing a voice activation mode, embodiments of the present system may obtain ECGI and their associate symptoms and/or context without having to look at, use touch inputs, etc., to enter data which may be difficult, if not impossible (and/or legal), to do when, for example, the patient is mobile such as when driving a car, etc.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device or memory, such as one or more of the memories 126, 191, 196 or other memory coupled to a controller.

The program and/or program portions contained in the one or more memories may configure the controller to implement the methods, operational acts, and functions disclosed herein. The memories may be distributed, for example between the clients and/or servers, or local, and the controller, where additional processors may be provided, may also be distributed or may be singular. The memories may be implemented as electrical, magnetic or optical memory, or any combination of these or other types of storage devices. Moreover, the term "memory" should be construed broadly enough to encompass any information able to be read from or written to an address in an addressable space accessible by the controller. With this definition, information accessible through a network such as the network 108 is still within the memory, for instance, because the controller may retrieve the information from the network 108 for operation in accordance with embodiments of the present system.

The controller(s) is/are operable for providing control signals and/or performing operations in response to input signals from the user input device (e.g., UI) as well as in response to other devices of a network, such as the sensors 114 and executing instructions stored in the one or more memories. The one or more controllers may include one or more of a microprocessor, an application-specific and/or general-use integrated circuit(s), a logic device, etc. Further, the one or more controllers may be a dedicated processor for performing in accordance with the present system and/or may be a general-purpose processor wherein only one of many functions operates for performing in accordance with the present system. The one or more controllers may operate utilizing a program portion, multiple program segments, and/or may be a hardware device utilizing a dedicated or multi-purpose integrated circuit.

Further variations of the present system would readily occur to a person of ordinary skill in the art and are encompassed by the following claims. For example, as previously discussed regarding embodiments, the MCTU may send ECGI to the MS. Alternatively, the MCTU may send all ECGI to the MS (e.g., every 5 minutes the ECGI of the last 5 minutes is sent to the phone). In these embodiments or other embodiments wherein ECGI is available, when the patient informs the MCTU about the occurrence of a symptom, the MS may create the ECGI directly and enter the VRM to start a voice dialogue with the patient to describe the symptom (e.g., to formulate the URI). As described, the patient may inform the MCTU and/or the MS about the occurrence of a symptom. In addition, the software (process, controller, etc.) of the MCTU and/or the MS may detect abnormalities (e.g. an abnormal heart rhythm) indicated by the ECGI. When an abnormality is detected, the MS may start a voice dialogue with the patient to know the context of the patient (e.g., What were you doing? Did you experience any symptoms? Are you OK now?).

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended spirit and scope of the present system as set forth in the claims that follow. In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming), and any combination thereof;
f) hardware portions may be comprised of one or both of analog and digital portions;
g) any of the disclosed devices, features and/or portions thereof may be combined together or separated into further portions unless specifically stated otherwise;
h) no specific sequence of acts or steps is intended to be required unless specifically indicated; and
i) the term "plurality of' an element includes two or more of the claimed element, and does not imply any particular range of number of elements; that is, a plurality of elements may be as few as two elements, and may include an immeasurable number of elements.

## Claims

1. A mobile telemetry system (100), comprising:
a mobile telemetry unit (MTU) (102) including:
a sensor (114) for detecting a physiological signal; and
a controller (120) configured to:
communicate with a mobile station (MS) (104) to perform a clock synchronization with a clock of the MS (104);
obtain the physiological signal from the sensor and form physiological information;
determine whether a time stamped transmit information request (TS-XReq) including time stamp information (TS) was received from the MS (104); and
generate and transmit physiological information which corresponds in time with the received TS when it is determined that the TS-XReq was received from the MS (104).

2. The mobile telemetry system of claim 1, wherein the controller (120) is a first controller (120), the system further comprising the MS (104), the MS (104) having a second controller (190) configured to transmit the TS-XReq to the MTU (102) and to control a voice recording mode (VRM) to obtain symptom information corresponding to the TS of the TS-XReq and form corresponding user response information (URI).

3. The mobile telemetry system of claim 2, wherein the second controller (190) is configured to associate the TS with the URI to form time stamped user response information (TS-URI).

4. The mobile telemetry system of claim 1, wherein the controller (120) is a first controller (120), the system further comprising the MS (104), the MS (104) having a second controller (190) configured to generate the time stamp information (TS) and the TS-XReq in response to a user interaction at the MS (104) and to transmit the TS-XReq to the MTU (102).

5. The mobile telemetry system of claim 4, wherein the second controller (190) is configured to control a voice recording mode (VRM) to obtain symptom information corresponding to the TS of the TS-XReq and form corresponding user response information (URI).

6. The mobile telemetry system of claim 5, wherein the second controller (190) is configured to associate the TS with the URI to form time stamped user response information (TS-URI).

7. The mobile telemetry system of claim 6, wherein the second controller (190) is further configured to associate corresponding patient identification information (ID) with the generated TS-URI to form ID-TS-URI.

8. The mobile telemetry system of claim 7, wherein the first controller (120) is configured to transmit the physiological information to the MS (104), and wherein the second controller (190) is further configured to transmit the ID-TS-URI and the physiological information to a clinical service center (CSC) (106).

9. The mobile telemetry system of claim 1, wherein the controller (120) is a first controller (120), the system further comprising a second controller (190) configured to control a voice recording mode (VRM) to obtain symptom information corresponding to the TS of the TS-XReq and form corresponding user response information (URI) associated with the TS as time stamped user response information (TS-URI).

10. The mobile telemetry system of claim 9, further comprising the MS (104), wherein the MS (104) is a first MS and the sensor is a first sensor, the system further comprising a second MS, the second MS having the second controller, wherein the second controller is further configured to receive the TS-XREQ, the first MS having a third controller and a second sensor for detecting a user interaction, wherein the third controller is configured to determine whether a user interaction is detected by the second sensor and transmit the TS-XREQ to the second MS and to the MTU (102) when the user interaction is detected.

11. The mobile telemetry system of claim 10, wherein the second controller (190) is further configured to associate corresponding patient identification information (ID) with the generated TS-URI to form ID-TS-URI.

12. The mobile telemetry system of claim 11, wherein the first controller (120) is configured to transmit the physiological information to the MS (104), and wherein the second controller (190) is further configured to transmit the ID-TS-URI and the physiological information to a clinical service center (CSC) (106).

13. The mobile telemetry system of claim 1, wherein the sensor (114) is a first sensor (114), the MTU (102) further comprising a second sensor (118) for detecting a user interaction, wherein the controller (120) is configured to generate and transmit, when it is determined that the second sensor has detected the user interaction: physiological information which corresponds in time with when the user interaction was detected by the second sensor; a TS which corresponds in time with when the user interaction was detected by the second sensor; and a TS activate voice recording mode (TS-VRM) command configured to activate a voice recording mode (VRM) of a mobile station to capture user response information (URI).

14. A mobile cardiac telemetry unit (MCTU) (102), comprising:
a cardiac sensor (114) for detecting cardiac signals; and
a controller (120) configured to:
obtain cardiac information from the cardiac sensor and form electrocardiograph information (ECGI);
determine whether a user request has been generated and form time stamp information (TS) when it is determined that the user request has been generated, the TS substantially corresponding with a time that the user request was determined to be generated;
form an activate voice recording mode command (AVRM) configured to activate a voice recording mode (VRM) of a mobile station (MS) (104) to capture user response information (URI);
generate electrocardiograph information (ECGI), which corresponds in time with the TS; and
transmit the TS, the AVRM and the ECGI.

15. The MCTU of claim 14 , wherein the cardiac sensor (114) is a first sensor, the system further comprising a second sensor (118) for detecting a user interaction, wherein the controller (120) is configured to:
determine whether the user interaction is detected by the second sensor (118), the user interaction indicating the user request; and
transmit the TS, the AVRM and the ECGI when it is determined that the second sensor has detected the user interaction.
